# EUROPEAN PATENT APPLICATION

(11) **EP 2 845 597 A1**
(43) Date of publication of application: **11.03.2015**
(21) Application number: 13183789.0
(22) Date of filing: 10.09.2013
(51) Int. Cl.: A61K 31/722, A61K 47/10, A61P 31/10

(54) **Hydroxypropyl chitosan for the treatment of onychomycosis**

(71) Applicant: POLICHEM S.A., 1526 Luxembourg (LU)
(72) Inventor: Mailland, Federico, CH-6900 Lugano (CH); Caserini, Maurizio, I-22100 Como (IT); Ceriani, Daniela, I-21050 Besano (VA) (IT)
(74) Representative: Pistolesi, Roberto

(57) **Abstract**

The present invention is directed to a method to treat onychomycosis by topically administering a composition which contains hydroxypropyl chitosan as the sole active ingredient and, in particular, by administering a nail topical composition consisting essentially of:
a) hydroxypropyl chitosan,
b) water,
c) at least a lower alkanol.

## Description

The present invention is directed to a method to treat onychomycosis by topically administering a composition which contains hydroxypropyl chitosan as the sole active ingredient.

### BACKGROUND OF THE INVENTION

Onychomycosis is a difficult to eradicate fungal infection of the nails which requires the use of antifungal agents either by oral route or by a topical application of compositions suitable to remain adherent to the nail surface for a period of time sufficient to release the antifungal agents to the nail, which is the site of action.

The oral pharmacological treatment is done by terbinafine, which is actually considered as the golden standard for onychomycosis worldwide, and is reported to achieve a complete cure in 38% of patients. Alternatives to oral terbinafine are itraconazole and fluconazole, also administered by oral route, reportedly less effective. None of those drugs, terbinafine, itraconazole or flucanazole, is devoid of rare but serious, sometimes fatal adverse events (Ajit C, Suvannasankha A, Zaeri N, Munoz SJ, Terbinafine-associated hepatotoxicity. Am J Med Sci. 2003; 325:292-5; Sl⌀rdal L, Spigset O. Heart failure induced by non-cardiac drugs. Drug Saf. 2006; 29:567-86).

To avoid the risk of severe adverse events by oral antifungal agents, topical treatments have been developed, including ciclopirox, amorolfine and tioconazole: among them ciclopirox is the most effective agent, achieving 12.7% or 5.8% cure rate according to the different vehicles used.

WO02/07683A1 discloses water soluble chitosans as film forming agent of nail varnish compositions, which are per se devoid of any antimycotic activity, but act synergistically with antimycotic agents to enhance their antimycotic activity, thus are suitable to be ingredients of topical antimycotic compositions with enhanced antimycotic properties.

A nail solution including ciclopirox as active ingredient, hydroxypropyl chitosan as film forming agent, ethyl acetate as penetration enhancer, cetostearyl alcohol as plasticizer, ethanol and water as solvent system, done according to the matter disclosed in WO02/07683A1, after daily application for 48 weeks plus 12 weeks of follow up, achieved a cure rate of 12.7% of patients, being superior both to the vehicle including hydroxypropyl chitosan, ethyl acetate, cetostearyl alcohol, ethanol and water (cure rate 1.3%) and to a commercial solution of ciclopirox without hydroxypropyl chitosan (cure rate 5.8%) (Baran R, Tosti A, Hartmane I et al. An innovative water soluble biopolymer improves efficacy of ciclopirox nail lacquer in the management of onychomycosis. J Eur Acad Dermatol Veneorol, 2009, 23:773-781).

In very stringent pivotal studies, done to document the efficacy of topical antimycotic compositions to treat patients with onychomycosis, it is needed to document the superiority of the test drug against its own vehicle, which has the same quali-quantitative formulation of the ingredients other than the active. In those studies, the vehicle always results as devoid of any activity, patients of vehicle groups achieving the endpoint "complete cure" ranging around 0.0% and 0.9% in patients of ciclopirox study (Gupta AK, Fleckman P, Baran R. Ciclopirox nail lacquer topical solution 8% in the treatment of toenail onychomycosis. J Am Acad Dermatol. 2000;43:70-80), 1.3% in patients with the aforementioned hydroxypropyl chitosan, ethyl acetate, cetostearyl alcohol, ethanol and water solution (Baran R, Tosti A, Hartmane I et al. An innovative water soluble biopolymer improves efficacy of ciclopirox nail lacquer in the management of onychomycosis. J Eur Acad Dermatol Veneorol, 2009, 23:773-781), 0.8% and 0.0% in a topical terbinafine/amorolfine study (Elewski B, Ghannoum MA, Mayser P et al. Efficacy, safety and tolerability of topical terbinafine nail solution in patients with mild-to-moderate toenail onychomycosis: results from three randomized studies using double-blind vehicle-controlled and open-label active-controlled designs. J Eur Acad Dermatol Veneorol, 2011, DOI: 10.1111/j.1468-3083.2011.04373.x.

It has now been surprisingly found that a simple nail solution of hydroxypropyl chitosan as film forming agent and a proper solvent system is effective in the treatment of onychomycosis despite the lack of any antimycotic agent into the composition, when it is administered for at least 6 months to at least one year, to patients in need of a treatment for onychomycosis.

### DESCRIPTION OF THE INVENTION

An object of the present invention is a method to treat onychomycosis by topically administering a composition which contains hydroxypropyl chitosan as the sole active ingredient, together with pharmaceutically acceptable excipients and/or adjuvants.

A nail topical composition which may be used in the method of the present invention consists essentially of:
a) hydroxypropyl chitosan,
b) water
c) at least a lower alkanol.

The composition to be used in the method according to the present invention does not need to contain an active ingredient to be effective in eradicating the fungus and ameliorating the appearance of the nail in a substantive proportion of patients. Moreover, the composition to be used in the method according to the present invention does not need the presence of any penetration enhancer to lead any antimycotic agent efficiently penetrate into and through the nail plate, with the clear advantage of preventing to expose patients and environment to an antifungal agent.

The composition to be used in the method of the present invention comprises hydroxypropyl chitosan, namely a water soluble film forming agent, as component a). Film forming agents are by definition (see e.g. DIN 55945 (12/1988)) components of a binder which are essential for forming a film, i.e. a thin layer or cover. The term "water soluble" means in this context that the film forming agent is fully compatible with water so that at 20°C one part of the film forming agent is soluble in 100 parts or less, preferably 50 parts or less, more preferably 30 parts or less, most preferably 10 parts or less of water.

The amount of the component a) is preferably in the range from 0.1 to 10% w/w, more preferably from 0.3 to 2% w/w, of the total composition.

The composition to be used in accordance with the present invention further comprises water as component b). The preferred amount of component b) in accordance with the present invention is from 10 to 40% w/w, more preferably from 18 to 30% w/w, of the total composition.

The composition to be used in accordance with the present invention further comprises a lower alkanol or a mixture of lower alkanols as a solvent as component c). The lower alkanol is preferably a C₁-C₄- alkanol and may be selected from ethanol, propanol, isopropanol, buthanol.

Preferably, the total amount of lower alkanol used in combination with water present in the composition in accordance with the present invention is such to provide acceptable drying times of the formulation once applied to the nails. An acceptable drying time, i.e. the time taken to be dry by touch, is less than about two minutes. Component c) is usually employed in an amount suitable in order to impart the above noted properties. It is preferred that the component c) is present in the composition in accordance with the present invention in an amount from 45 to 95% w/w, more preferably from 60 to 80% w/w, of the total composition.

The composition to be used in accordance with the present invention is preferably applied on the surface of the infected fingernails and/or toenails in a thin layer, by means of a brush, or of a spatula or any applicator, and left to dry for 0.5-2 minutes. After applying the composition, washing the nails should be avoided for at least 6 hours, more preferably for at least 12 hours, most preferably for 24 hours, in order to avoid loss of medication due to nails' exposure to water. The application should be repeated once daily, preferably at bed time, after shower and drying, for a minimum period of 6 months to one year. The application period of at least 6 months is recommended for treatment of infected fingernails, the period of at least one year is recommended for treatment of infected toenails.

According to a preferred embodiment, the composition to be used in the method of the present invention consists of:
a) hydroxypropyl chitosan in amount from 0.3% to 1.0% by weight,
b) water in amount from 18% to 40% by weight,
c) ethanol in amount from 60% to 80% by weight.

The composition to be used in the method of the present invention is illustrated, but not limited to, the following examples. All amounts in % are w/w %.

### EXAMPLE 1

Nail lacquer formulations having the following compositions by weight are prepared:

| hydroxypropyl chitosan | 2.0% | 1.5% | 1.0% | 0.5% | 0.3% |
|---|---|---|---|---|---|
| purified water | 38.0% | 28.5% | 22.0% | 19.5% | 29.7% |
| ethanol | 60.0% | 70.0% | 77.0% | 80.0% | 70.0% |

The formulations are prepared by using a suitable closed vessel provided with a stirrer. To this vessel are added ethanol, deionized water and hydroxypropyl chitosan and the resulting mixture is stirred until dissolution.

The obtained nail lacquer compositions have a clear and homogeneous appearance and are perfectly transparent and colorless even after prolonged storage.

### COMPARATIVE EXAMPLE 2

Comparative nail lacquer formulations are prepared:

| | | |
|---|---|---|
| terbinafine HCl | 5% | 10.0% |
| hydroxypropyl chitosan | 0.3% | 0.3% |
| purified water | 24.7% | 19.7% |
| ethanol | 70.0% | 70.0% |

The formulations are prepared by using a suitable closed vessel provided with a stirrer. To this vessel are added ethanol, deionized water and terbinafine HCl to form a mixture. Thereafter, hydroxypropyl chitosan is added and the resulting mixture is stirred until dissolution.

The obtained nail lacquer compositions have a clear and homogeneous appearance and are perfectly transparent and colorless even after prolonged storage.

### EXAMPLE 3

A multicentre, randomized, double-blind within frequency of administration, vehicle controlled, dose-finding, parallel-group study was completed in patients with mild-to-moderate dermatophyte onychomycosis (distal lateral subungual onychomycosis, defined as 25%-60% clinical involvement of the target toenail, without dermatophytomas or matrix/lunula involvement) randomized to apply for 52 weeks one of the following treatment regimens:
1) 10% terbinafine HCl once daily as per the Comparative Example 2 for the whole treatment length (P-3058 10% o.d., n= 93),
2) 10% terbinafine once daily as per the Comparative Example 2 for the first month, followed by 10% terbinafine once weekly until the end of treatment period, (P-3058 10% o.w., n=91),
3) 5% terbinafine HCl once daily as per the Comparative Example 2 (P-3058 5% o.d., n=94),
4) Hydroxypropyl chitosan once daily or once weekly, at the concentration of 0.3% as per the Example 1, namely not containing any terbinafine nor any other antifungal agent (n= 92).

The treatment period was followed by 24-weeks of follow-up. The investigation was aimed at evaluating the effect of the different doses of the investigational product P-3058 compared to the vehicle in the treatment of onychomycosis at the end of follow-up (week 76).

The primary efficacy endpoint was the proportion of patients achieving "Responder rate" at the end of the wash-out period (week 76), defined as composite parameter of ≤10% clinical involvement of the target toenail and mycological cure (negative microscopic KOH examination and negative culture). The key secondary efficacy endpoint was the proportion of patients achieving "Complete cure" defined as composite parameter of 0% clinical involvement of the target toenail and mycological cure (negative microscopic KOH examination and negative culture) at different time points during the treatment phase as well as during the wash-out period.

Overall, 370 patients were included in the efficacy analysis (MITT population). At baseline, the percentage of the affected target toenail area was in average 40.7% (min: 14, max: 70).

The results were as follows: concerning primary efficacy endpoint, at the end of follow-up (week 76), the rate of responder patients were: 16.13% in P-3058 10% o.d., 15.96% in P-3058 5% o.d., 23.08% in P-3058 10% o.w., 20.65% in vehicle group. As a conclusion, the efficacy of the hydroxypropyl chitosan solution (= vehicle) on primary parameter was superior to that of both 10% and 5% terbinafine solutions, and was inferior only to terbinafine 10% once weekly solution.

As far as the key secondary efficacy endpoint was concerned, at the end of follow-up (week 76), the rates of complete cured patients were: 8.6% in P-3058 10% o.d., 7.45% in P-3058 5% o.d., 10.99% in P-3058 10% o.w., 6.52% in vehicle group.

Although the rate of complete cure for vehicle was inferior in this investigation to all the active formulations containing terbinafine in different dosage regimens, nonetheless the complete cure for the hydroxypropyl chitosan vehicle was superior to that of other vehicles reported in the literature of onychomycosis, which do not contain hydroxypropyl chitosan, or contain it combined with other ingredients, like ethyl acetate and cetostearyl alcohol, as reported in the following table:

| Study | Vehicle composition | Rate of complete cure |
|---|---|---|
| Gupta* I | Gantrez, isopropyl alcohol | 0.9% |
| Gupta* II | Gantrez, isopropyl alcohol | 0.0% |
| Baran** | hydroxypropyl chitosan, ethyl acetate, cetostearyl alcohol, ethanol, water | 1.3% |
| Elewski^{£} 24 wk | DDAIP, benzyl alcohol, polyvinylpyrrolidone, ethanol | 0.8% |
| Elewski^{£} 48 wk | DDAIP, benzyl alcohol, polyvinylpyrrolidone, ethanol | 0.0% |
| Example 1 | hydroxypropyl chitosan, ethanol, water | 6.52% |

| | | |
|---|---|---|
| * Gupta AK et al. J Am Acad Dermatol. 2000; 43:70-80 ** Baran R et al. J Eur Acad Dermatol Veneorol, 2009, 23:773-781 ^{£} Elewski B et al. J Eur Acad Dermatol Veneorol, 2011, DOI: 10.1111/j.1468-3083.2011.04373.x | | |

As a conclusion, both in the primary and in the secondary efficacy endpoints the group of patients treated with the vehicle alone achieved appreciable results in terms of efficacy to treat onychomycosis.

## Claims

1. A composition which contains hydroxypropyl chitosan as the sole active ingredient together with pharmaceutically acceptable excipients and/or adjuvants, for use in the treatment of onychomycosis.

2. The composition for use according to claim 1, **characterized by** consisting essentially of:
a) hydroxypropyl chitosan,
b)water,
c)at least a lower alkanol.

3. The composition for use according to claim 2, **characterized in that** the amount of hydroxypropyl chitosan is from 0.1 to 10% by weight of the total composition.

4. The composition for use according to claim 3, **characterized in that** the amount of hydroxypropyl chitosan is from 0.3 to 2% by weight of the total composition.

5. The composition for use according to claim 2, **characterized in that** amount of water is from 10 to 40% by weight of the total composition.

6. The composition for use according to claim 5, **characterized in that** amount of water is from 18 to 30% by weight of the total composition.

7. The composition for use according to claim 2, **characterized in that** said at least lower alkanol is a C₁-C₄-alkanol.

8. The composition for use according to claim 7, **characterized in that** said C₁-C₄-alkanol is selected from ethanol, propanol, isopropanol, butanol.

9. The composition for use according to claim 8, **characterized in that** said C₁-C₄-alkanol is ethanol.

10. The composition for use according to claim 2, **characterized in that** the amount of said at least lower alkanol is from 45 to 95% by weight of the total composition.

11. The composition for use according to claim 2, **characterized in that** the amount of said at least lower alkanol is from 60 to 80% by weight of the total composition.

12. The composition for use according to claim 1, **characterized by** consisting of:
a) hydroxypropyl chitosan in amount from 0.3% to 1.0% by weight,
b) water in amount from 18% to 40% by weight,
c) ethanol in amount from 60% to 80% by weight.

13. The composition for use according to any of the preceding claims, **characterized in that** the recipient of such a treatment is a human.

14. The composition for use according to any of the preceding claims, **characterized in that** it is applied to fingernails and/or toenails once daily.

15. The composition for use according to any of the preceding claims, **characterized in that** it is applied to fingernails and/or toenails for at least 6 months to at least one year.
